# EUROPÄISCHE PATENTANMELDUNG

(11) **EP 3 871 640 A1**
(43) Veröffentlichungstag der Anmeldung: **01.09.2021**
(21) Anmeldenummer: 21020107.5
(22) Anmeldetag: 26.02.2021
(51) Int. Cl.: A61F 5/10, A61F 5/058, A61F 5/01

(54) **ORTHESE FÜR DAS DAUMENSATTELGELENK**

(30) Priorität: 26.02.2020 DE 102020105011
(71) Anmelder: Matzen, Miklas, 21382 Brietlingen (DE)
(72) Erfinder: Matzen, Miklas, 21382 Brietlingen (DE)
(74) Vertreter: Völger, Karl Wolfgang

(57) **Zusammenfassung**

Die vorliegende Erfindung betrifft eine Orthese (1) für das Daumensattelgelenk (CMP), umfassend:
- eine Schale (101), die vom proximalen Handwurzelgelenk (RP) nach distal bis zum Daumengrundgelenk (MCP) verläuft,
- einen an der Schale (101) befestigten Verschluss (103) im Bereich des proximalen Handwurzelgelenks (RP), welcher am proximalen Handwurzelgelenk (RP) zirkulär vollkommen umschließend fixierbar ist,
- eine Schiene (107), die von der Schale (101) bis zum Daumenendgelenk (IP) verläuft, und
- eine Halterung (109), die am Ende der Schiene (107) vorgesehen ist und am distalen Ende des ersten Daumenknochens (PPI) zirkulär vollkommen umschließend fixierbar ist,
dadurch gekennzeichnet, dass lateral des Daumengrundgelenks (MCP) ein mechanisches Gelenk (105) angeordnet ist, dessen mechanischer Drehpunkt parallel zum anatomischen Drehpunkt liegt, wobei das mechanische Gelenk (105) die Schale (101) mit der Schiene (107) reversibel lösbar verbindet.

Die vorliegende Erfindung bezieht sich ferner auf die Verwendung der erfindungsgemäßen Orthese (1) nach einem der Ansprüche 1 bis 8 zur Ausübung einer Zugkraft auf das Daumensattelgelenk (CMP) und/oder auf das Daumengrundgelenk (MCP) zur Entlastung der Gelenkflächen.

## Beschreibung

Die vorliegende Erfindung betrifft eine Orthese für das Daumensattelgelenk zur Linderung der Beschwerden einer Daumensattelgelenkserkrankung aus dem degenerativen, entzündlichen und rheumatischen Formenkreis. Ferner betrifft die Erfindung die Verwendung dieser Orthese.

Das Daumensattelgelenk (CMP), lat. Articulatio carpometacarpalis pollicis, ist die gelenkige Verbindung zwischen dem großen Vieleckbein, lat. Os trapezium, in der Handwurzel und dem Mittelhandknochen des Daumens, lat. Os metacarpale I. Es ist das einzige Gelenk der fünf Grundgelenke, Articulationes carpometacarpales, das frei beweglich ist. Mögliche Bewegungen sind Flexion, Extension, Abduktion, Adduktion, Rotation und Opposition. Das Daumensattelgelenk (CMP) wird vor allem für die Gegenüberstellung (Opposition) des Daumens benötigt und hat daher eine zentrale Bedeutung für das Greifen.

Bei der Arthrose des Daumensattelgelenks (CMP), konkret als Rhizarthrose bezeichnet, handelt es sich um eine Erkrankung infolge derer es zu unspezifischen Abnutzungserscheinungen einhergehend mit einer Verringerung der Gleitschicht des Knorpelgelenks bis hin zu dessen vollständiger Auflösung kommen kann. Damit verbunden sind zunehmende Schmerzen, Druckempfindlichkeit bis hin zu einer aktivierten Arthrose (entzündliche Form der Arthrose). Des Weiteren wird der Kapsel-Band-Apparat sekundär geschädigt und geschwächt.

Bisher aus dem Stand der Technik bekannte Behandlungsmethoden der Rhizarthrose stellen das Daumensattelgelenk (CMP) ruhig, wodurch als schmerzhaft empfundene Bewegungen mehr oder weniger effektiv unterbunden werden. Alternativ kann ein nicht einstellbares, nur bei Beugung aktives Auseinanderziehen des Daumensattelgelenks (CMP) herbeigeführt werden.

So wird in DE 30 06 362 A1 eine Vorrichtung aus zwei offenen, am Unterarm befestigten Stegen beschrieben, bei der unter anderem der Daumen in einer zum Schienenende verlaufenden Hülse so eingeführt ist, dass die gesamte Hand einschließlich des Handgelenks bewegungsunfähig arretiert ist.

DE 35 19 493 A1 offenbart eine Vorrichtung zum Ruhigstellen des Daumensattelgelenks (CMP), bei welcher der Daumen und die Hand zumindest teilweise von einem festen Formkörper umschlossen fixiert sind. Durch die anatomische Ausformung der Schiene verbleibt die Hand in ihrer natürlichen Lage und bewirkt so die Ruhigstellung des Daumensattelgelenks (CMP) als auch des Daumengrundgelenks (MCP), lat. Articulatio metacarpophalangea.

Auch in WO 2011/110420 A1 wird eine Vorrichtung beansprucht, deren Aufgabe eine spezifische und vollständige Immobilisierung des Daumensattelgelenks (CMP) erreicht und so die schmerzhafte Bewegung der von der Arthrose beeinträchtigten Gelenke sicher unterbindet.

Die bekannten Vorrichtungen aus dem Stand der Technik haben den Nachteil, dass durch die Ruhigstellung des Gelenks der Kapsel-Band-Muskelapparat in kurzer Zeit erschlafft und infolge dessen eine fortschreitende Einschränkung in der Beweglichkeit einhergeht mit einer Verschlimmerung der Schmerzsymptome.

In DE 20 2014 103 362 U1 wird eine Vorrichtung aus Kunststoff beschrieben, welche dadurch gekennzeichnet ist, dass sie anatomisch angepasst und lösbar am Handgelenk fixiert ist. Sie verläuft lateral über den Handballen und schließt korkenzieherähnlich am ersten Daumengelenk ab. Diese Vorrichtung hat den Nachteil, dass keine Verstellmöglichkeit gegeben ist und ein Auseinanderziehen des Daumensattelgelenks (CMP) nur bei Beugung des Daumenendgelenks (IP), lat Articulatio interphalangea, erfolgt, was einen Nutzen für Patienten, welche z.B. einen Rollstuhl über die Antriebsräder selber antreiben müssen, nicht gewährleisten kann.

Weitere Nachteile der bekannten Vorrichtungen bestehen darin, dass die gewünschte Ruhigstellung nur dann erreichbar ist, wenn die mitunter auch plastisch verformbare und damit anatomisch angepasste Schiene die zu arretierenden Körperteile fest umschließt. Dadurch wird die Blutzirkulation beeinträchtigt und infolge punktuell wirkenden Drucks werden Empfindlichkeiten und Scheuerstellen hervorgerufen. Zudem wird das Ausführen auch nur leichter körperlicher Betätigung infolge der Ruhigstellung nahezu komplett unterbunden und der aus Daumen und Mittelfinger bildbare Pinzettengriff deutlich erschwert oder gar unmöglich.

In Erkenntnis dieser Probleme weicht DE 20 2012 009 952 U1 von den vorerwähnten Prinzipien ab und stellt das Daumensattelgelenk (CMP) nicht nur ruhig, sondern spreizt dieses durch eine in die anatomisch angepasste Orthese eingeklebte flügelförmige Pelotte aus einem nachgiebig gepolsterten Material. Ein Druck auf die Daumenspitze, wie er insbesondere beim Pinzettengriff wirkt, wird so über die anatomisch ausgeformte Orthese auf die Mittelhand abgeleitet, was gleichsam den Druck auf die Pelotte erhöht und die aufeinander reibenden Gelenkschalen zunehmend expandiert.

Allerdings birgt diese Vorrichtung den Nachteil, dass das Daumensattelgelenk (CMP) fest umschlossen in einer Schiene ruht. Durch den gegen die Schiene ausgelösten Druck, der durch die eingeklebte Pelotte noch erhöht wird, kann es beim Tragen der Vorrichtung zu punktuellen Belastungen bis hin zu Druckstellen kommen. Das Anpassen der Orthese bereitet darüber hinaus nicht unerhebliche Schwierigkeiten, weil die Pelotte nur exakt ausgerichtet das Auseinanderdrücken des Gelenkes bewirkt, andernfalls den Schmerz der Grunderkrankung sogar verstärkt. Die hier beschriebene Vorrichtung ist zudem nur wirksam, wenn das Daumenendgelenk (IP) bewegt wird.

Angesichts der vorstehend beschriebenen Nachteile und Probleme des Standes der Technik ist es Ziel der vorliegenden Erfindung, die vorstehenden Nachteile zu überwinden und eine Orthese bereitzustellen, durch die das arthrotisch veränderte Daumensattelgelenk (CMP) dynamisch entlastet wird, bei gleichzeitiger Forderung des Kapsel-Band-Muskelapparats.

Diese Aufgabe wird erfindungsgemäß in einem ersten Aspekt der vorliegenden Erfindung durch eine Orthese (1) für das Daumensattelgelenk (CMP) gelöst, umfassend
- eine Schale (101), die vom proximalen Handwurzelgelenk (RP) nach distal bis zum Daumengrundgelenk (MCP) verläuft,
- einen an der Schale (101) befestigten Verschluss (103) im Bereich des proximalen Handwurzelgelenks (RP), welcher am proximalen Handwurzelgelenk (RP) zirkulär vollkommen umschließend fixierbar ist,
- eine Schiene (107), die von der Schale (101) bis zum Daumenendgelenk (IP) verläuft, und
- eine Halterung (109), die am Ende der Schiene (107) vorgesehen ist und am distalen Ende des ersten Daumenknochens (PPI) zirkulär vollkommen umschließend fixierbar ist,
dadurch gekennzeichnet, dass lateral des Daumengrundgelenks (MCP) ein mechanisches Gelenk (105) angeordnet ist, dessen mechanischer Drehpunkt parallel zum anatomischen Drehpunkt liegt, wobei das mechanische Gelenk (105) die Schale (101) mit der Schiene (107) reversibel lösbar verbindet.

Die erfindungsgemäße Orthese (1) ist zur Befestigung an einer menschlichen Hand vorgesehen. Abhängig von der rechten oder der linken Hand ist die erfindungsgemäße Orthese (1) spiegelbildlich ausgebildet.

Die Schale (101) wird in ihrer konkreten Form individuell an die Hand des Patienten angepasst und liegt am Handballen passgenau an. Sie verläuft vom proximalen Handwurzelgelenk (RP) nach distal bis zum Daumengrundgelenk (MCP).

Der Verschluss (103) im Bereich des proximalen Handwurzelgelenks (RP), lat. Articulatio radiocarpalis, welcher am proximalen Handwurzelgelenk (RP) zirkulär vollkommen umschließend fixierbar ist, kann in der einfachsten Ausführungsform aus einem festen Band bestehend, das um das Handgelenk geschlungen wird. Des Weiteren ist ein Verschluss (103) mit einem Klett-/Flauschband mit einem Umlenker erfindungsgemäß möglich.

Die Schiene (107) dient einerseits dazu, die Halterung (109) für das distale (körperferne) Ende des ersten Daumenknochens (PPI), lat. Phalanx proximalis I, aufzunehmen. Andererseits erstreckt sich die Schiene (107) in etwa parallel zum ersten Daumenknochens (PPI). Der Begriff "Schiene" umfasst jegliches langgestreckte Bauteil, das dazu geeignet ist, diese Funktion zu übernehmen. Die Schiene (107) im Sinne der vorliegenden Erfindung schließt daher auch Stäbe, Rohre und dergleichen ein.

Die Halterung (109) umschließt das distale Ende des ersten Daumenknochens (PPI) zirkulär vollkommen und ist damit reversibel fixierbar. In der einfachsten Ausführungsform kann auch diese Halterung aus einem schalenförmigen Teil und einem festen Band bestehen, das um den Daumen geschlungen wird. Des Weiteren ist ein Verschluss (103) mit einem Klett-/Flauschband mit einem Umlenker erfindungsgemäß möglich.

Die vorliegende Erfindung zeichnet sich dadurch aus, dass lateral (d.h. auf der Höhe) des Daumengrundgelenks (MCP) ein mechanisches Gelenk (105) angeordnet ist, dessen mechanischer Drehpunkt parallel zum anatomischen Drehpunkt liegt. Durch diese Anordnung des mechanischen Gelenks (105) wird die Beweglichkeit der Hand, bzw. des Daumens trotz anlegender Orthese (1) gewährleistet. Ferner zeichnet sich die erfindungsgemäße Orthese (1) dadurch aus, dass das mechanische Gelenk (105) die Schale (101) mit der Schiene (107) reversibel lösbar verbindet.

Die Formulierung "reversibel lösbar" bedeutet, dass die Schiene (107) in dem mechanischen Gelenk (105) so aufgenommen ist, dass sie sowohl befestigt als auch wieder gelöst werden kann, um sie auf den Daumen des Patienten einzustellen. Zudem erleichtert dies das An- und Ausziehen der Orthese (1).

Das mechanische Gelenk (105) hat die Möglichkeit, dem anatomischen Gelenk in Flexion (Beugung) und Extension (Streckung) zu folgen. Es begrenzt also nicht die Bewegungen des anatomischen Gelenks.

Durch die Beweglichkeit der erfindungsgemäßen Orthese (1) am Daumengrundgelenk (MCP) ist eine Haltung in jeder Stellung dieses Gelenks möglich. Dadurch wird ein Abbau des Kapsel-Band-Muskelapparat durch eine stetige, aber gleichzeitig schonende Beanspruchung verhindert.

Eine Beugung des Daumenendgelenks (IP) zur Entastung, wie in DE 20 2014 103 362 U1 beschrieben, ist mit der erfindungsgemäßen Orthese (1) nicht mehr notwendig, wodurch eine dauerhafte Entlastung des Gelenks gewährleistet wird.

Zudem kann je nach Schwere der Tätigkeit der Zug auf das Daumengrundgelenk (MCP) reduziert oder verstärkt werden. Außerdem wird das Daumengrundgelenk (MCP) nicht vollständig ruhiggestellt.

In einer bevorzugten Ausführungsform der erfindungsgemäßen Orthese (1) ist das mechanische Gelenk (105) dazu ausgelegt, die Schiene (107) reversibel verschiebbar aufzunehmen, so dass der Abstand zwischen Schale (101) und Halterung (109) reversibel verstellbar ist.

Die Formulierung "reversibel verschiebbar" bedeutet, dass die Schiene (107) so in dem mechanische Gelenk (105) gelagert ist, dass sie (in gewissen Grenzen) hinein und herausgeschoben werden kann, das heißt nach distal oder proximal verschoben werden kann, um so den Abstand zwischen Schale (101) und Halterung (109) reversibel zu verstellen. Auf diese Weise kann eine Zugkraft auf das Daumengrundgelenk (MCP) je nach Bedarf eingestellt werden.

Das mechanische Gelenk (105) übernimmt folglich hat die Aufgabe, die Schiene (107) einzufahren und herauszuschieben und diese in einer bestimmten Position dann zu fixieren. Dieses Fixieren ist beispielsweise durch eine Vorrichtung in der Art eines BOA-Verschlusses (wie er z.B. bei Schuhen verwendet wird) möglich, welcher nicht zieht, sondern schiebt.

Durch diese Verstellung der im Bereich des Daumengrundgelenks (MCP) angebrachten erfindungsgemäßen Orthese (1) kommt es zu einer geringen Spaltbildung des Daumensattelgelenks (CMP), da eine Spannung zwischen der am proximalen Handwurzelgelenk (RP) anliegenden Schale (101) mit Verschluss (103) und der Halterung (109) unterhalb des Daumenendgelenks (IP) angelegt wird. Dadurch wird ein Reiben der Gelenkflächen des Daumensattelgelenks (CMP) verhindert oder zumindest reduziert, was mit einer spürbaren Verringerung der Schmerzen einhergeht. Zudem bekommen die Gelenkflächen so die Möglichkeit, ein Ersatzgewebe zu bilden, welches eine neue Schutzschicht des Knochens darstellt. Dabei wird das Daumensattelgelenk (CMP) um bis zu 0,25 mm gestreckt.

Ein weiterer Vorteil ist, dass durch die weiterhin mögliche Bewegung der Gelenke die Produktion der Gelenkschmiere, lat. Synovia, angeregt wird, welche z.B. den restlichen vorhandenen Knorpel der Gelenkflächen mit Nährstoffen versorgt und als Gleitmittel dient.

Aus therapeutischen Gründen kann es in einer Weiterbildung sinnvoll sein, wenn das mechanische Gelenk (105) in seinem Winkelbereich begrenzbar ist. Mit diesem Winkelbereich wird der Bewegungsbereich des Daumensattelgelenks (CMP) und des Daumengrundgelenks (MCP) begrenzt.

Damit kann beispielsweise bei einer Hypermobilität des Daumengrundgelenks (MCP) die Flexion begrenzt werden, um weiterhin einen guten Kraftverlauf zu gewährleisten.

Ebenfalls aus therapeutischen Gründen kann es angezeigt sein, das mechanische Gelenk (105) in einer vorbestimmten Position zu sperren, beispielsweise über Nacht.

Da das Daumensattelgelenk (CMP) das einzige Gelenk der fünf Grundgelenke ist, das frei beweglich ist, ist es bevorzugt, diese freie Beweglichkeit in vollem Umfang dadurch sicherzustellen, dass das mechanische Gelenk (105) drehbar gelagert ist. Bei Bedarf kann auch ein in der Flexion (Beugung) und Extension (Streckung) begrenzendes mechanisches Gelenk (105) mit denselben Funktionen benutzt werden.

Eine spezielle Ausführungsform sieht vor, dass die Schale (101) mit der Schiene (107) reversibel lösbar verbindbar ist, in dem das mechanische Gelenk (105) in einem vorgegebenen Bewegungsfreiraum herausziehbar ist.

Um ein Verrutschen der Schale (101) am proximalen Handwurzelgelenk (RP) zu vermeiden, weist die Schale (101) zumindest einen Druckpunkt (101a) distal des distalen Gelenkkopfs der Elle (CU), lat. Caput ulnae, oder distal des Griffelfortsatzes der Speiche (PSR), lat. Processus styloideus radii, auf. Der Druckpunkt (101a) kann insbesondere als Pelotte ausgebildet sein, die in die Schale (101) eingearbeitet ist.

Um ein Verrutschen der Schale (101) noch weiter zu verhindern, kann auch in dem Verschluss (103) ein Druckpunkt, insbesondere in Form einer Pelotte, vorgesehen sein, der distal des distalen Gelenkkopfs der Speiche (CR), lat. Caput radii, angeordnet ist. Ferner kann die Schale (101) mit rutschfesten Polstern versehen werden (z.B. aus Kautschuk).

Nach einer anderen Weiterbildung der erfindungsgemäßen Orthese (1) weist die Schale (101) eine halbe bis vollständige Umschließung proximal des Daumengrundgelenks (MCP) auf. Hierdurch wird die Parallelität des anatomischen Gelenkdrehpunkts und des Drehpunkts des mechanischen Gelenks (105) gewährleistet.

Es ist bevorzugt, wenn die erfindungsgemäße Orthese (1) ferner eine Anzeigevorrichtung zur Überwachung einer Zugkraft aufweist. Hiermit kann einerseits sichergestellt werden, dass eine therapeutisch maximal zulässige Zugkraft nicht überschritten wird. Andererseits wird gewährleistet, dass der Patient nicht zu wenig Zugkraft ausübt und somit die Wirkung der erfindungsgemäßen Orthese (1) nicht oder nicht vollständig nutzt.

Die vorstehend genannte Aufgabe wird erfindungsgemäß in einem zweiten Aspekt der vorliegenden Erfindung durch eine Verwendung der erfindungsgemäßen Orthese (1) gelöst zur Ausübung einer Zugkraft auf das Daumensattelgelenk (CMP) und/oder auf das Daumengrundgelenk (MCP) zur Entlastung der Gelenkflächen.

Wie vorstehend schon erwähnt, kann durch das Anlegen einer Zugkraft das Daumensattelgelenk (CMP) entlastet und die Bildung von Ersatzgewebe im Gelenk gefördert werden. Zudem kann direkt nach dem Anlegen der erfindungsgemäßen Orthese (1) und dem Einstellen des richtigen Zugs eine direkte Reduzierung der Schmerzen beim Patienten erreicht werden.

Die vorstehend genannte Aufgabe wird erfindungsgemäß in einem dritten Aspekt der vorliegenden Erfindung durch eine Verwendung der erfindungsgemäßen Orthese (1) gelöst zur Linderung von Beschwerden einer Erkrankung des Daumensattelgelenks (CMP) aus dem degenerativen, entzündlichen und rheumatischen Formenkreis.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1:: eine schematische Darstellung einer menschlichen Hand von der Handinnenfläche her betrachtet mit der erfindungsgemäßen Orthese 1 und
- Fig. 2:: eine schematische Darstellung der in Figur 1 dargestellten menschlichen Hand vom Handrücken her betrachtet mit der erfindungsgemäßen Orthese 1.

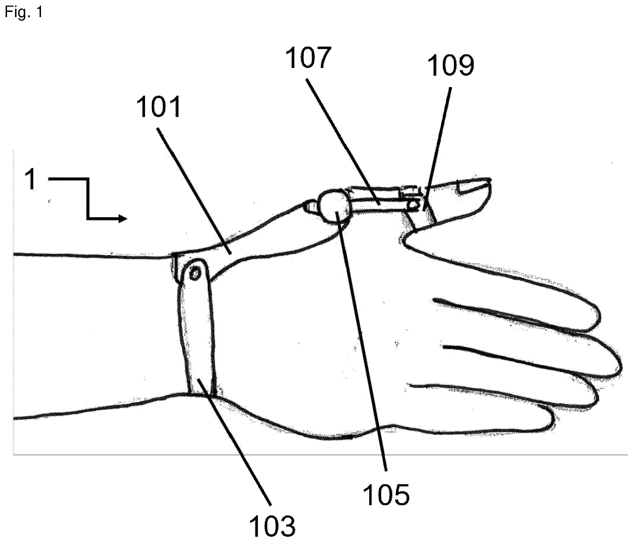

In Figur 1 wird eine menschliche Hand schematisch von der Handinnenfläche her (palmar) betrachtet dargestellt, wobei die erfindungsgemäße Orthese 1 angebracht ist. Die Schale 101 liegt auf dem Handballen auf und wird in dieser konkreten Ausführungsform mit dem Verschluss 103 von der dorsalen (rückseitigen), radialen (speichenseitig) und palmaren (handflächenseitigen) Seite am proximalen Handwurzelgelenk RP befestigt. Das Handgelenk wird somit zirkulär vollkommen umschlossen.

Der Verschluss 103 besteht hier aus einem Band, kann aber je nach Beschaffenheit und Bedarf auch variiert werden. Das Befestigen geschieht beispielsweise durch eine Haken-Ösen-Verbindung oder ein Klett-/Flauschband.

Auf der Höhe des Daumengrundgelenks MCP ist das mechanische Gelenk 105 zu erkennen, in das die Schiene 107 eingebracht ist. Am anderen Ende der Schiene 107 ist die Halterung 109 vorgesehen, die am distalen Ende des ersten Daumenknochens PPI ansetzt und diesen zirkulär vollkommen umschließt. Die Befestigung der Halterung 109 kann analog der Befestigung des Verschlusses 103 erfolgen.

In einer bevorzugten Ausführungsform weist die Halterung 109 eine U-förmige Halbschale auf, die aus einem der hier angegebenen Materialien gefertigt ist. Diese kann, entweder von dorsal oder palmar angebracht, zirkulär geschlossen werden. Die U-förmige Halbschale befindet sich unterhalb des Daumenendgelenks IP und gibt Druck unterhalb des ersten Daumenknochens PPI auf, so dass ein Verrutschen der U-förmigen Halbschale nach distal verhindert wird.

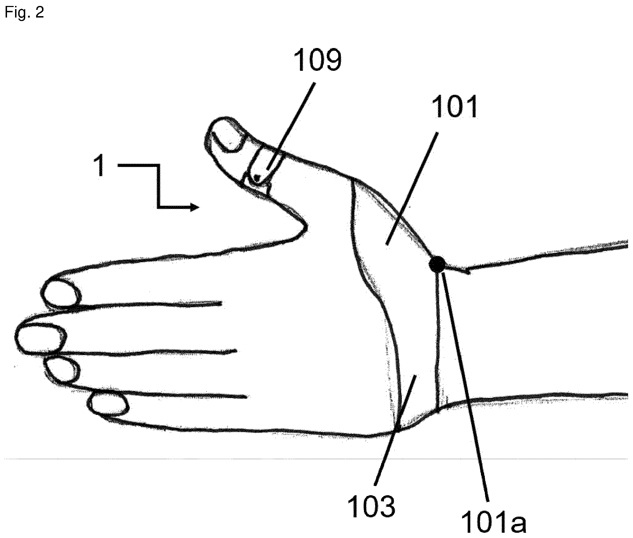

Figur 2 stellt die in Figur 1 gezeigte menschliche Hand schematisch vom Handrücken her (dorsal) betrachtet dar, wobei die andere Seite der erfindungsgemäßen Orthese 1 zu sehen ist. In dieser Darstellung wir lediglich schematisch der Druckpunkt 101a gezeigt, der sich distal des distalen Gelenkkopfs der Elle CU befindet.

Die erfindungsgemäße Orthese 1 kann je nach Bedingungen aus unterschiedlichen Materialien gefertigt werden. Bevorzugt besteht sie aus einem erstarkten thermoplastischen Kunststoff, der das Handgelenk teilweise umschließt. Bevorzugt ist ein Niedertemperatur-Thermoplast oder ein Faserverbundwerkstoff.

In einer weiteren bevorzugten Ausgestaltung besteht die Schiene 101 aus einem gelochten Kunststoff, um der Haut das Atmen zu erleichtern.

Die Schiene 107 wird bevorzugt aus einem Niedertemperatur-Thermoplast gefertigt.

### Bezugszeichenliste

- 1: Orthese
- 101: Schale
- 101a: Druckpunkt
- 103: Verschluss
- 105: mechanisches Gelenk
- 107: Schiene
- 109: Halterung

- CMP: Daumensattelgelenk
- CR: Gelenkkopf der Speiche
- CU: Gelenkkopf der Elle
- IP: Daumenendgelenk
- MCP: Daumengrundgelenk
- PPI: erster Daumenknochen
- PSR: Griffelfortsatzes der Speiche
- RP: proximalen Handwurzelgelenk

## Patentansprüche

1. Orthese (1) für das Daumensattelgelenk (CMP), umfassend:
- eine Schale (101), die vom proximalen Handwurzelgelenk (RP) nach distal bis zum Daumengrundgelenk (MCP) verläuft,
- einen an der Schale (101) befestigten Verschluss (103) im Bereich des proximalen Handwurzelgelenks (RP), welcher am proximalen Handwurzelgelenk (RP) zirkulär vollkommen umschließend fixierbar ist,
- eine Schiene (107), die von der Schale (101) bis zum Daumenendgelenk (IP) verläuft, und
- eine Halterung (109), die am Ende der Schiene (107) vorgesehen ist und am distalen Ende des ersten Daumenknochens (PPI) zirkulär vollkommen umschließend fixierbar ist,
**dadurch gekennzeichnet, dass** lateral des Daumengrundgelenks (MCP) ein mechanisches Gelenk (105) angeordnet ist, dessen mechanischer Drehpunkt parallel zum anatomischen Drehpunkt liegt, wobei das mechanische Gelenk (105) die Schale (101) mit der Schiene (107) reversibel lösbar verbindet.

2. Orthese (1) nach Anspruch 1, wobei das mechanische Gelenk (105) dazu ausgelegt ist, die Schiene (107) reversibel verschiebbar aufzunehmen, so dass der Abstand zwischen Schale (101) und Halterung (109) reversibel verstellbar ist.

3. Orthese (1) nach Anspruch 1 oder 2, wobei das mechanische Gelenk (105) in seinem Winkelbereich begrenzbar ist.

4. Orthese (1) nach einem der Ansprüche 1 bis 3, wobei das mechanische Gelenk (105) in einer vorbestimmten Position gesperrt werden kann.

5. Orthese (1) nach einem der Ansprüche 1 bis 4, wobei das mechanische Gelenk (105) drehbar gelagert ist.

6. Orthese (1) nach einem der Ansprüche 1 bis 5, wobei die Schale (101) mit der Schiene (107) reversibel lösbar verbindbar ist, indem das mechanische Gelenk (105) in einem vorgegebenen Bewegungsfreiraum herausziehbar ist.

7. Orthese (1) nach einem der Ansprüche 1 bis 6, wobei die Schale (101) zumindest einen Druckpunkt (101a) distal des distalen Gelenkkopfs der Elle (CU) oder distal des Griffelfortsatzes der Speiche (PSR) aufweist.

8. Orthese (1) nach einem der Ansprüche 1 bis 7, wobei die Schale (101) eine halbe bis vollständige Umschließung proximal des Daumengrundgelenks (MCP) aufweist.

9. Orthese (1) nach einem der Ansprüche 1 bis 8, wobei diese ferner eine Anzeigevorrichtung zur Überwachung einer Zugkraft aufweist.

10. Verwendung der Orthese (1) nach einem der Ansprüche 1 bis 9 zur Ausübung einer Zugkraft auf das Daumensattelgelenk (CMP) und/oder auf das Daumengrundgelenk (MCP) zur Entlastung der Gelenkflächen.

11. Verwendung der Orthese (1) nach einem der Ansprüche 1 bis 9 zur Linderung von Beschwerden einer Erkrankung des Daumensattelgelenks (CMP) aus dem degenerativen, entzündlichen und rheumatischen Formenkreis.
